# EUROPEAN PATENT SPECIFICATION

(11) **EP 0 666 746 B1**
(45) Date of publication and mention of the grant of the patent: **04.07.2001**
(21) Application number: 94927309.8
(22) Date of filing: 01.09.1994
(51) Int. Cl.: A61K 31/56, C07C 49/105, C07C 49/293, A61K 31/565

(54) **$g(D)5-ANDROSTENES USEFUL FOR PROMOTING WEIGHT MAINTENANCE OR WEIGHT LOSS AND TREATMENT PROCESS**
Delta - 5 - Androstene, die das Halten des Gewichts oder den Gewichtsverlust fördern und Behandlungsverfahren
$g(D)5-ANDROSTENES UTILES POUR FAVORISER LE MAINTIEN OU LA PERTE DE POIDS ET METHODE DE TRAITEMENT

(30) Priority: 02.09.1993 US 123151
(43) Date of publication of application: 16.08.1995
(73) Proprietor: HUMANETICS CORPORATION, Chanhassen, Minnesota 55317 (US)
(72) Inventor: LARDY, Henry, A., Madison, WI 53705 (US); REICH, Ieva, L., Madison, WI 53711 (US); YONG, Wei, Washington Boro, NJ 07882 (US)
(74) Representative: Patentanwälte Hauck, Graalfs, Wehnert, Döring, Siemons
(86) International application number: US9409852
(87) International publication number: WO9506472

(56) References cited:
- WO-A-92/03925
- US-A- 5 028 631

## Description

### Field of the Invention

The invention relates to steroids selected from the group of Δ5-androstenes for use as weight control agents, especially for weight gain prevention, weight reduction and treatment of obesity.

The steroid dehydroepiandrosterone (DHEA) is believed to stimulate various biological responses including (i) inducing the synthesis of various thermogenic enzymes which are effective for regulating metabolism and thereby promoting weight control without affecting caloric intake, and (ii) inducing an increase in the production of the sex hormones androgen and estrogen.

The ability of DHEA to promote weight control is believed to be mediated through enhanced thermogenesis (conversion of foodstuffs to heat energy rather than chemical energy such as ATP and/or triacylglycerides). The thermogenic effect mediated by DHEA is believed to result from the ability of DHEA to stimulate the synthesis of thermogenic enzymes including mitochondrial glycerol 3-phosphate dehydrogenase (G3P-DH) and cytosolic malic enzyme (ME). Such enzymes tend to reduce the efficiency of energy metabolism within the body.

Unfortunately, DHEA is generally considered to be ineffective as a weight controlling therapeutic agent because the dosage necessary to achieve weight control frequently produces significant adverse side-effects including substantial increases in the concentration of sex hormones.

From PCT /US91/06147 a treatment process for promoting weight loss is known which employs a substituted Δ5-androstene. The steroids disclosed therein can be considered effective for stimulating the desired biological response while being ineffective for the undesired biological responses, e.g. induction of the synthesis of sex hormones.

Further therapeutic agents possessing the weight control characteristic of DHEA without the adverse side-effect of stimulating the synthesis of sex hormones would be extremely useful. In addition, the effectiveness of such a therapeutic agent could be significantly enhanced if the agent possessed an increased weight control activity relative to DHEA.

### Summary of the invention

Δ5-androstenes providing the desired beneficial biological response without the undesired biological response include:

A5-Androstene-3β-acetoxy-7, 16, 17-trione (5)

Δ5-Androstene-3β, 16α-dihydroxy-7, 17-dione (6)

Δ5-Androstene-3β-propionoxy-16β-acetoxy-7, 17-dione (7)

Δ5-Androstene-3β, 7α, 17β-triol-16-one (8)

Δ5-Androstene-3β, 17β-diol-7, 16-dione (9)

Δ5-Androstene-3β, 16α, 17β-triol, 7-one (10)

and derivatives thereof wherein one or more of the hydroxyl or keto substituents is a group convertible thereto by hydrolysis.

Examples of such hydrolyzable groups include hydroxyl groups esterified with an acid selected from the group consisting of (i) normal or branched, saturated or unsaturated C₂₋₂₂ aliphatic acids, (ii) C₇₋₁₂ aromatic acids, (iii) C₃ or larger dicarboxylic acids in which only one of the carboxyl groups is esterified to the hydroxyl group(s) on the steroid, or (iv) inorganic acids such as sulfuric and phosphoric.

### Detailed Description of the Invention Including a Best Mode

The Δ5-androstenes identified below possess a unique combination of properties including the ability to promote weight control without affecting appetite and without stimulating the production of sex hormones.

A5-Androstene-3β-acetoxy-7, 16, 17-trione (5)

Δ5-Androstene-3β, 16α-dihydroxy-7, 17-dione (6)

Δ5-Androstene-3β-propionoxy-16β-acetoxy-7, 17-dione (7)

Δ5-Androstene-3β, 7α, 17β-triol-16-one (8)

Δ5-Androstene-3β, 17β-diol-7, 16-dione (9)

Δ5-Androstene-3β, 16α, 17β-triol, 7-one (10)

and derivatives thereof wherein one or more of the hydroxyl or keto substituents is a group convertible thereto by hydrolysis.

Examples of such hydrolyzable groups include hydroxyl groups esterified with an acid selected from the group consisting of (i) normal or branched, saturated or unsaturated C₂₋₂₂ aliphatic acids, (ii) C₇₋₁₂ aromatic acids, (iii) C₃ or larger dicarboxylic acids in which only one of the carboxyl groups is esterified to the hydroxyl group(s) on the steroid, and (iv) inorganic acids such as sulfuric and phosphoric.

These steroids may also be administered as a carbamate or other such derivative capable of releasing the specified steroid within the intestinal tract, blood and/or body tissue. The desired biological activity is a function of the steroid moiety. Derivations of a moiety may serve a variety of beneficial functions including stabilization of the steroid, flavoring or obscuring the natural flavor of the steroid, or affecting the rate of absorption of the steroid.

### Synthesis

### (5) Δ5-Androstene-3β-acetoxy-7,16,17-trione

Δ5-Androstene-3β-acetoxy-7,16,17-trione (5) may be synthesized from commercially available DHEA acetate by sequentially converting:

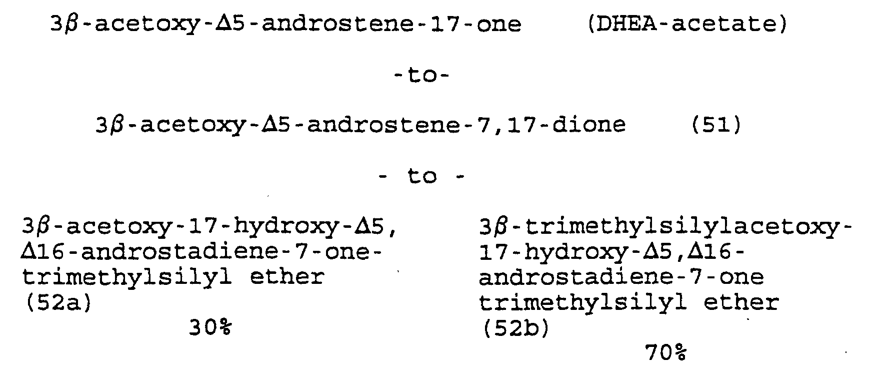

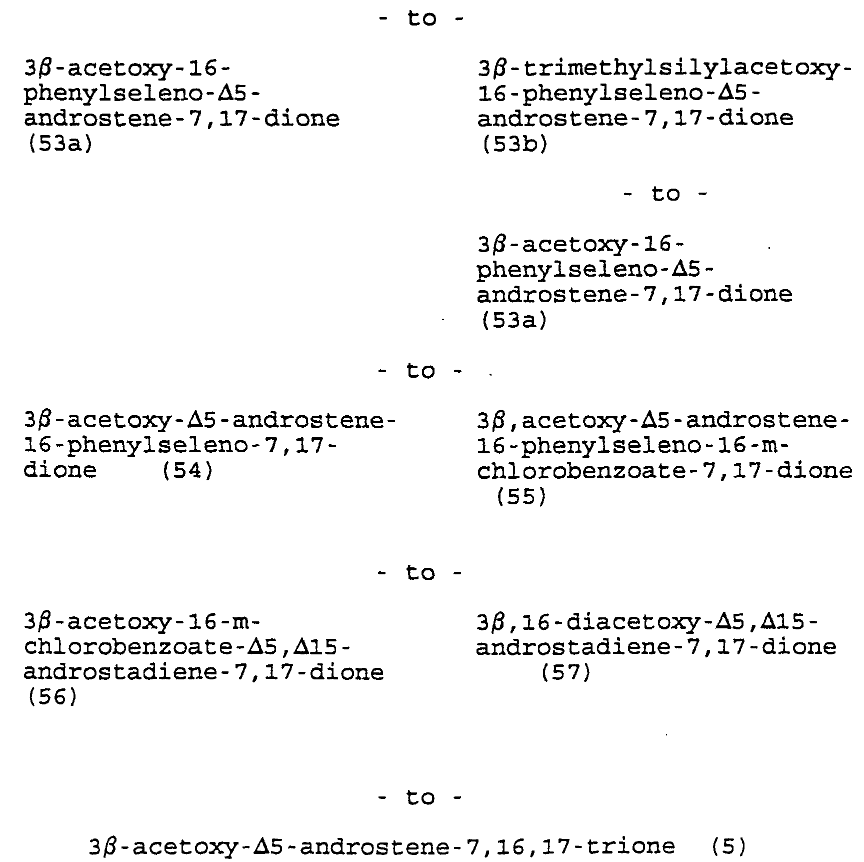

3β-Acetoxy-Δ5-androstene-7,17-dione (51) can be synthesized from 3β-acetoxy-Δ5-androstene-17-one (DHEA-acetate) by reacting DHEA-acetate with the oxidizing agent CrO₃ in accordance with the procedure outlined in Fieser, L.F., Jour. Am. Chem. Soc., vol. 75, pp 4386-4394 (1953).

A mixture of 70% 3β-(trimethylsilyl)acetoxy-17-hydroxy-Δ5,Δ16-androstadiene-7-one trimethylsilyl androstadiene-7-one trimethylsilyl ether (52a) may be synthesized from the 3β-acetoxy-Δ5-androstene 7,17-dione (51) by reacting (51) with lithium diisopropyl amide in the presence of trimethylchlorosilane in a suitable solvent such as tetrahydrofuran at -78°C.

3β-Acetoxy-16-phenylseleno-Δ5-androstene-7,17-dione (53a) and 3β-(trimethylsilyl)acetoxy-16-phenylseleno-Δ5-androstene-7,17-dione (53b) may be synthesized from 3β-acetoxy-17-hydroxy-Δ5,Δ16-androstadiene-7-one trimethylsilyl ether (52a) and 3β-(trimethylsilyl)acetoxy-17-hydroxy-Δ5,Δ16-androstadiene-7-one trimethylsilyl ether (52b) respectively by reacting the mixture of (52a) and (52b) with benzeneselenenyl chloride in the presence of pyridine and a suitable solvent such as tetrahydrofuran at -78°C.

The carbon silylated acetate attached to the C₃ carbon atom on the (53b) fraction of the (53) mixture may be desilylated so as to convert (53b) to (53a) within the (53) mixture by treating the (53) mixture with tetra-n-butylammonium fluoride in a suitable solvent system such as etherdichloromethane-tetrahydrofuran-water.

3β,16-Diacetoxy-Δ5-androstene-16-phenylseleno-7,17-dione (54) may be synthesized from the 3β-acetoxy-16-phenylseleno-Δ5-androstene-7,17-dione (53a) by submitting (53a) to the seleno-Pummerer reaction outlined in Ikota, N.; Ganem, B. Jour. Org. Chem., vol. 43, pp. 1607-1608 (1978). Briefly, the 3β-acetoxy-16-phenylseleno-Δ5-androstene-7,17-dione (53a) is sequentially reacted with m-chloroperbenzoic acid and acetic anhydride in a suitable solvent. 3β-acetoxy-Δ5-androstene-16-phenylseleno-16-m-chlorobenzoate-7,17-dione (55) is also synthesized during this reaction.

3β,16-diacetoxy-Δ5,Δ15-androstadiene-7,17-dione (57) may be synthesized from 3β,16-diacetoxy-Δ5-androstene-16-phenylseleno-7,17-dione (55) by oxidative dehydrogenation. The 3β-acetoxy-Δ5-androstene-16-phenylseleno-16-m-chlorobenzoate-7,17-dione (54) present with (55) produces 3β-acetoxy-16-m-chlorobenzoate-Δ5,Δ15-androstadiene-7,17-dione (56) which may be separated by chromatography if desired.

3β-acetoxy-Δ5-androstene-7,16,17-trione (5) may be synthesized from the 3β-acetoxy-Δ5,Δ15 androstadiene-16-m-chlorobenzoate-7,17-dione (56) and/or 3β,16-diacetoxy-Δ5,Δ15-androstadiene-7,17-dione (57) by treating them with triethylamine in methanol.

### (6) Δ5-Androstene-3β,16α-dihydroxy-7,17-dione (7-keto-16α-hydroxy DHEA)

3β,16α-dihydroxy-Δ5-androstene-7,17-dione can be synthesized by the following sequence from DHEA propionate. DHEA propionate can be made by simple esterification of DHEA.

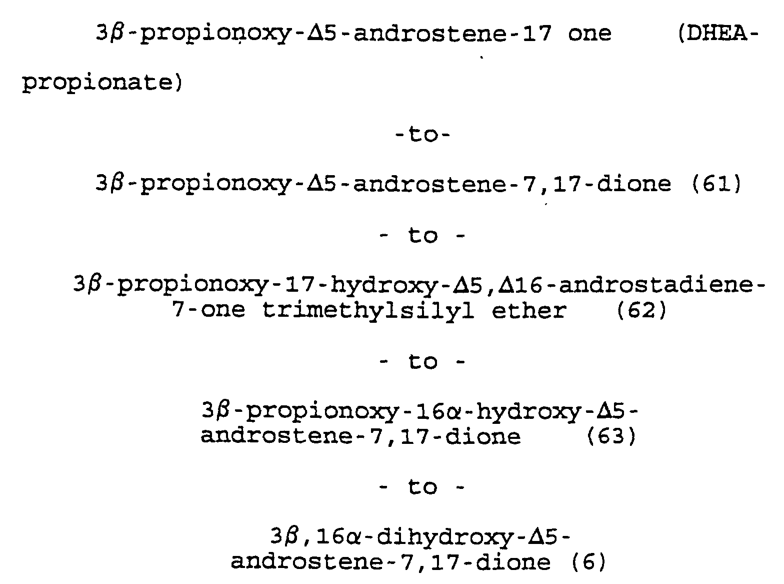

3β-Propionoxy-Δ5-androstene-7,17-dione (61) (NMR set forth at table eight) can be synthesized from 3β-propionoxy-Δ5-androstene-17-one (DHEA-propionate) by reacting the DHEA-propionate with the oxidizing agent CrO₃ in accordance with the procedure outlined in Fieser, L.F., Jour. Am. Chem. Soc., vol. 75, pp 4386-4394 (1953).

3β-Propionoxy-17-hydroxy-Δ5,Δ16-androstadiene-7-one trimethylsilyl ether (62) may be synthesized from the 3β-propionoxy-Δ5-androstene-7,17-dione (61) by reacting (61) with lithium diisopropyl amide in the presence of trimethylchlorosilane in a suitable solvent such as tetrahydrofuran at -78°C.

3β-Propionoxy-16α-hydroxy-Δ5-androstene-7,17-dione (63) may be synthesized from the 3β-propionoxy-17-hydroxy-Δ5,Δ16-androstadiene-7-one trimethylsilyl ether (62) by oxidation with m-chloroperbenzoic acid in tetrahydrofuran followed by treatment with a 1N HCl solution.

The final desired product 3β,16α-dihydroxy-Δ5-androstene-7,17-dione (6) may then be synthesized from the 3β-propionoxy-16α-hydroxy-A5-androstene-7,17-dione (63) by treatment with sulfuric acid in methanol.

### (7) Δ5-Androstene-3β-propionoxy,16β-acetoxy-7,17-dione

### (7-Keto-16β-acetoxy DHEA propionate)

Δ5-Androstene-3β-propionoxy-16β-acetoxy-7,17-dione (7) can be synthesized from Δ5-androstene-3β-propionoxy-16α-hydroxy-7,17-dione (63) produced in accordance with the reaction sequence set forth above. The A5-androstene-3β-propionoxy-16α-hydroxy-7,17-dione is treated in accordance with the Mitsunobu conditions set forth in Hughes, D. L.; Reamer, R.A.; Bergan, J.J.; Grabowski, E.J.J. Jour Am. Chem. Soc., vol. 110, pp 6487-6491 to invert the configuration and acetylate the 16 hydroxy group.

### (8) Δ5-Androstene-3β,7α,17β-triol-16-one

Δ5-Androstene-3β,7α,17β-triol-16-one (8) can be synthesized from A5-androstene-3β,16α-dihydroxy-17-one diacetate (81). The Δ5-androstene-3β,16α-dihydroxy-17-one diacetate (81) starting material can be synthesized in accordance with the procedure set forth in Numazawa, M. and Osawa, Y. Steroids, vol. 32, p 519 (1978).

Δ5-Androstene-3β,16α-diacetoxy-7-bromo-17-one (82) can be synthesized from the Δ5-androstene-3β,16α-diacetoxy-17-one (81) by reacting Δ5-androstene-3β,16α-diacetoxy-17-one (81) with a brominating agent, such as Dibromantin (1,3-dibromo-5,5-dimethylhydantoin). The Δ5-androstene-3β,16α-diacetoxy-7-bromo-17-one (82) is unstable and must be used immediately in the next step of the process.

The Δ5-androstene-3β,16α-diacetoxy-7-bromo-17-one (82) contains an isomeric mixture of 7α-bromo and 7β-bromo isomers. The isomeric mixture, may be equilibrated to 7α-bromo in accordance with the method described for equilibriating a cholesterol derivative in Confalone, P.N., Kulesha, I.D., and Uskokovic, M.R. Jour. Orq. Chem., vol. 46, pp 1030-1032 (1981). Briefly, the isomeric mixture is contacted with cold anhydrous LiBr and shielded from light to convert the product from an isomeric mixture of 7α and 7β to predominently 7α.

Δ5-Androstene-3β, 16α-diacetoxy-7α-hydroxy-17-one (83) may be synthesized from the Δ5-androstene-3β,16α-diacetoxy-7α-bromo-17-one (82) by reacting the Δ5-androstene-3β,16α-diacetoxy-7α-bromo-17-one (82) with a mixture of glacial acetic acid and powdered silver acetate at room temperature in a suitable solvent, such as a mixture of methylene chloride and acetone. Also produced in this reaction is a 20% yield of Δ5-androstene-3β,7α,16α-triacetoxy-17-one.

Δ5-Androstene-3β,7α,17β-trihydroxy-16-one (8) can be synthesized from the Δ5-Androstene-3β,16α-diacetoxy-7α-hydroxy- 17-one (83) by dissolving the Δ5-Androstene-3β,16α-diacetoxy-7α-hydroxy- 17-one (83) in a room temperature methanol solution of K₂CO₃ and stirring the solution for two hours. The alkaline solution enolizes the 17-keto to form the more stable 17-hydroxy-16-one combination. The Δ5-androstene-3β,7α,17-trihydroxy-16-one (8) may then be isolated by filtering to remove the insoluble salt, evaporating the methanol in vacuo, purifying and remove extracting the compound by chromatography. The steroid crystallizes from a hot methanol/ether solution upon cooling.

### (9) Δ5-Androstene-3β,17β-diol-7,16-dione

Δ5-Androstene-3β,17β,-diol-7,16-dione (9) can be synthesized from 3β-propionoxy-16α-hydroxy-Δ5-androstene-7,17-dione (63) by treating 3β-propionoxy-16α-hydroxy-Δ5-androstene-7,17-dione (63) with a 5% aqueous sodium carbonate solution in methanol.

### (10) Δ5-Androstene-3β,16α,17β-triol,7-one

Δ5-Androstene-3β,16α,17β-triol-7-one (10) can be synthesized from 3β,16α,17β-triacetoxy-Δ5-androstene by reacting the 3β,16α,17β-triacetoxy-Δ5-androstene with the oxidizing agent CrO₃ in accordance with the procedure outlined in Fieser, L.F., Jour. Am. Chem. Soc., vol 75, pp 4386-4394 (1953) with subsequent hydrolysis of the acetyl groups.

Without intending to be unduly limited thereby, it is believed that the steroids identified above may be modified without loss of biological activity by esterifying one or more of the hydroxyl groups with any of a variety of organic acids and inorganic acids such as sulfuric or phosphoric acid.

### Treatment

A subject may be treated with the steroids specified herein by any of the commonly accepted practices including orally or by injection. While many factors affect the dose rate required to attain the desired biological response, treatment at a dosage rate of about 0.1 to 2 grams, preferably about 0.5 to 2 grams, of the steroid per 100 kilograms body weight per day should generally be effective for promoting weight control. A dose rate of less than 0.1 gram per 100 kilograms bodyweight per day is generally ineffective for preventing weight gain while a dose rate of greater than about 2 grams per 100 kilograms bodyweight per day increases the cost of treatment without providing a corresponding benefit in performance. The optimum dose rate to be administered to a subject is case specific as the optimum dose rate depends upon several factors including current body composition (percent fat), the desired effect (weight gain maintenance versus weight loss), eating habits of the individual (daily caloric intake), and the like. As would be expected, the dose rate provided to a subject for the purpose of promoting weight loss will be greater than that necessary to promote weight maintenance assuming identical caloric intake under each program.

Without intending to be limited thereby, we believe that the steroids specified herein are metabolic intermediates along the pathway to conversion of DHEA to an ultimate metabolite(s) which is actually responsible for mediating an enhanced production of thermogenic enzymes such as glycerol 3-phosphate dehydrogenase and malic enzyme.

A subject may be treated with one of the steroids specified herein on substantially any time schedule. It is believed that the steroids specified herein are effective for promoting weight control while the steroid itself is actively present within the body as well as while the increased concentration of thermogenic enzyme(s) induced by the steroid remain elevated. The in vivo life expectancy of the steroids and the thermogenic enzyme(s) induced thereby is not yet fully known. However, it is believed that the steroids themselves are not stored within the body and are removed and/or deactivated within days after administration. Accordingly, for optimum effectiveness, the subject under treatment should be treated every day or two. For reasons of convenience the subject under treatment may be treated less frequently, such as once a week, when less than maximum performance is acceptable.

As is apparent from the factors which affect dosage and dose rate, each particular subject should be carefully and frequently reviewed and the dosage and/or dose rate altered in accordance with the particular situation.

### Example VII (Steroid 5)

### Synthesis A5-Androstene-3β-acetoxy-7,16,17-trione

*(Step 1)* Into a 50 ml flask equipped with a magnetic stirrer and a water bath was placed 6.5 ml acetic anhydride, 23 ml acetic acid, 1.7 grams sodium acetate, and 2 grams DHEA acetate to form a first mixture. Into the first mixture was added 2 grams chromium trioxide over a thirty minute period to form a second mixture. The first mixture was maintained at a constant temperature of 56-58°C and continuously agitated during addition of the chromium trioxide.

*(Step 2)* The second mixture was maintained at 56-58°C and continuously agitated for an additional hour after which the second mixture was cooled and slowly poured under continuous agitation into 600 ml of ice water to form a precipitate. The flocculent precipitate was collected on a sintered glass funnel and washed with water until no longer green. After drying in vacuo over P₂O₅, the product was dissolved in methanol and recrystallized to yield substantially pure 3β-acetoxy-A5-androstene-7,17-dione (51) having a melting point of 184-185°C.

*(Step 3)* Into a second 50 ml round bottom flask equipped with a magnetic stirrer and retained within a dry-ice bath was placed 1.00 gram (2.90 mmoles) of the 3β-acetoxy-Δ5-androstene-7,17-dione (51) and 20 ml neat tetrahydrofuran to form a third mixture. The third mixture was placed under a N₂ atmosphere. Into the third mixture was added 1.07 ml (8.43 mmoles) trimethylchlorosilane to form a fourth mixture. The fourth mixture was maintained under the N₂ atmosphere and cooled to -78°C.

*(Step 4)* Into a third 25 ml round bottom flask equipped with a magnetic stirrer and retained within a dry-ice bath was placed 1.07 ml (7.66 mmoles) diisopropyl amine, 3.60 ml of a 1.94M solution of n-butyllithium in hexane (6.96 mmoles), and 4 ml of tetrahydrofuran to form lithium diisopropyl amide. The solution (LDA solution) was prepared under a N₂ atmosphere at -78°C. The LDA solution was warmed slightly to dissolve any solids therein and then added, under a N₂ atmosphere, to the fourth mixture via cannula to form a fifth mixture.

*(Step 5)* The fifth mixture was removed from the dry-ice bath and allowed to warm to room temperature for 15 minutes at which time 1.25 ml triethylamine was added to the fifth mixture to form a sixth mixture. Into a separatory funnel was placed 40 ml hexane, 40 ml of a saturated NaHCO₃ aqueous solution, and the sixth mixture. The organic phase was extracted with hexane, washed with a saturated NaCl aqueous solution, dried over Na₂SO₄ and the solvent removed to yield 1.42 grams of a dry organic solid. The organic solid was identified by NMR (CDCl₃) as an approximately 70:30 mixture of 3β-(trimethylsilyl)acetoxy-17-hydroxy-Δ5,Δ16-androstadiene-7-one trimethylsilyl ether (52b) and 3β-acetoxy-17-hydroxy-Δ5,Δ16-androstadiene-7-one trimethylsilyl ether (52a). The results of the NMR analysis are set forth in Tables One & Two.

*(Step 6)* Into a fourth 100 ml round bottom flask equipped with a magnetic stirrer and retained within a dry-ice bath was placed 2.85 mmoles of the organic solid obtained in Step 5, 20 ml neat tetrahydrofuran and 0.320 ml (4.00 mmole) neat pyridine to form a seventh mixture. The seventh mixture was cooled to -78°C and placed under a N₂ atmosphere. Into the seventh mixture was added 0.710 grams (3.71 mmoles) of benzeneselenenyl chloride in 4 ml of tetrahydrofuran to form an eighth mixture.

*(Step 7)* Into a separatory funnel was placed a cosolvent system of a 0.5 N HCl aqueous solution and dichloromethane. Into the cosolvent system was added the eighth mixture to extract the organic phase. The second organic phase was extracted by dichloromethane, sequentially washed with water and a saturated NaHCO₃ aqueous solution, dried over Na₂SO₄ and the solvent removed to yield 1.72 grams of an organic oil. The organic oil was separated by chromatography (100 grams silica eluted with hexane-ethyl acetate at 90:10 to 50:50 with 50 ml fractions) into 1.44 grams of first and second organic fractions (Fractions 14-17), diphenyl diselenide (Fractions 9-11) and unreacted 3β-acetoxy-Δ5-androstene-7,17-dione (51) (Fractions 18-19). The first and second organic fractions were identified by NMR as 3β-acetoxy-16^{*}-phenylseleno-Δ5-androstene-7,17-dione (53a) and 3β-(trimethylsilyl)acetoxy-16-phenylseleno-Δ5-androstene-7,17-dione (53b) respectively. The results of the NMR analysis are set forth in Tables Three and Four.

*(Step 8)* Into a fifth 100 ml round bottom flask equipped with a magnetic stirrer was placed 25 ml ether, 5 ml dichloromethane and 1.19 mmoles of the second organic fraction obtained in Step 7 (53b) to form a ninth mixture. Into the ninth mixture was added 8 ml of a 10% aqueous solution of potassium fluoride and 3 ml of a 1M tetra-n-butylammonium fluoride solution in tertrahydrofuran to form a tenth mixture. The tenth mixture was maintained at 25°C and continuously agitated for two hours after which the tenth mixture was poured into an ether-hexane-water solvent system to extract the organic phase. The extracted organic phase was washed twice with water, once with a saturated NaCl aqueous solution, dried over Na₂SO₄ and the solvent removed to yield 0.594 grams of 3β-acetoxy-16-phenylseleno-Δ5-androstene-7,17-dione (53a).

*(Step 9)* Into a sixth 100 ml round bottom flask equipped with a magnetic stirrer was placed 25 ml dichloromethane and 1.10 mmoles of 3β-acetoxy-16-phenylseleno-Δ5-androstene-7,17-dione (53a) to form an eleventh mixture. The eleventh mixture was cooled to 0°C and maintained under continuous agitation. Into the eleventh mixture was added 0.296 grams (1.2 mmoles) m-chloroperbenzoic acid (70%) followed five minutes later by 0.40 ml dimethylsulfide to form a thirteenth mixture. The thirteenth mixture was washed 3 times with 50 ml of a cold NaHCO₃ aqueous solution and filtered through Na₂SO₄.

*(Step 10)* Into a seventh 250 ml round bottom flask equipped with a magnetic stirrer was placed 1.2 ml acetic anhydride, 1.2 ml pyridine and the liquid phase of the thirteenth mixture to form a fourteenth mixture. The fourteenth mixture was continuously agitated at room temperature for an hour after which 60 ml of a saturated NaHCO₃ aqueous solution was added to form a fifteenth mixture having separate organic and inorganic layers. The fifteenth mixture was maintained under continuous agitation until bubbling ceased. The organic layer was separated from the inorganic layer by drawing off the organic layer in a separatory funnel. The separated organic layer was washed with a saturated NaHCO₃ aqueous solution, dried over Na₂SO₄ and the solvent removed to yield 0.713 grams of a dry organic solid. The organic solid was identified by NMR (CDCl₃) as a 90:10 mixture of 3β,16-diacetoxy-16-phenylseleno-Δ5-androstene-7,17-dione (55) and 3β-acetoxy-Δ5-androstene-16-phenylseleno-16-m-chlorobenzoate-7,17-dione (54). The results of an NMR analysis of the (55) fraction in the mixture are set forth in Table Five.

*(Step 11)* Into a round bottom flask equipped with a magnetic stirrer was placed 25 ml carbon tetrachloride and 1.10 mmoles of the 90:10 mixture of 3β,16-diacetoxy-16-phenylseleno-Δ5-androstene-7,17-dione (55) and 3β-acetoxy-Δ5-androstene-16-phenylseleno-16-m-chlorobenzoate-7,17-dione (54) to form a sixteenth mixture. Into the sixteenth mixture was added 0.12 ml pyridine, 10 mg benzeneseleninic acid, and 19 mg diphenyldiselenide to form a seventeenth mixture. The seventeenth mixture was maintained under vigorous agitation and 3.4 ml of a 15% H₂O₂ aqueous solution added to form an eighteenth mixture. The eighteenth mixture was agitated continuously for 45 minutes after which the eighteenth mixture was washed twice with a saturated NaHCO₃ aqueous solution and dried in vacuo to yield 0.390 grams of an organic solid. The organic solid was separated by chromatography (50 grams silica gel eluted with 50% hexane-ethyl acetate with 25 ml fractions) to yield 0.236 grams of an organic fraction (Fractions 9-10). The organic fraction was identified by NMR as pure 3β,16-diacetoxy-Δ5,Δ15-androstadiene-7,17-dione (57). The results of the NMR analysis are set forth in Table Six.

(Step 12) Into a round bottom flask equipped with a magnetic stirrer was placed 1.03 mmoles of 3β,16-diacetoxy-Δ5,Δ15-androstadiene-7,17-dione (57) and 15 ml methanol to form a nineteenth mixture. Into the nineteenth mixture was placed 0.500 ml triethylamine to form a twentieth mixture. The twentieth mixture was placed under a N₂ atmosphere and maintained under constant agitation for 16 hours. The twentieth mixture was then extracted between H₂O and CH₂Cl₂. The organic layer was washed twice with a saturated NaHCO₃ aqueous solution, and then dried and evaporated under reduced pressure to yield 0.281 grams of a yellow organic solid. The organic solid was identified by NMR as a mixture of organic compounds including about 65% 3β-acetoxy-Δ5-androstene-7,16,17-trione (5). The results of the NMR analysis are set forth in Table Seven.

### Example VIII (Steroid 6)

### Synthesis 3β,16α-dihydroxy-Δ5-androstene-7,17-dione

*(Step 1)* Into a round bottom flask equipped with a magnetic stirrer was placed 2.80 mmoles of 3β-propionoxy-17-hydroxy-Δ5,Δ16-androstadiene-7-one trimethylsilyl ether (62) synthesized in accordance with the procedure set forth in Example VII and identified by NMR (See Table Nine) and 40 ml of tetrahydrofuran to form a first mixture which was cooled to 0° in an ice bath. Into the first mixture was placed 2.95 mmoles of m-chloroperbenzoic acid (80-90%) to form a second mixture. The second mixture was warmed to 25°C and maintained under constant agitation for 10 min.

(Step 2) The second mixture was continuously agitated and 40 ml of a 1N HCl aqueous solution added to form a third mixture which was maintained under constant agitation for 20 min. The third mixture was partitioned between ether and water and separated by decanting the ether phase from the water phase. The ether phase was washed with a saturated NaHCO₃ aqueous solution, then a saturated NaCl aqueous solution, and dried in vacuo to yield an organic solid. The organic solid was purified by chromatography (140 gm of silica eluted with 60:40 to 70:30 ethyl acetate-hexane with 25 ml fractions) to give 0.586 gm of a first organic compound. The first organic compound was identified by NMR as 3β-propionoxy-16α-hydroxy-Δ5-androstene-7,17-dione (63). The results of the NMR analysis are set forth in Table Ten.

(Step 3) In a second round bottom flask equipped with a magnetic stirrer was placed 0.155 mmoles of 3β-propionoxy-16α-hydroxy-Δ5-androstene-7,17-dione (63) dissolved in 6 ml of methanol to form a third mixture. To this third mixture was added 1.5 ml of an aqueous solution of 6N sulfuric acid to form a fourth mixture which was maintained at 25°C for 18 hours. The fourth mixture was then partitioned between ethyl acetate and water and separated by decanting the ethyl acetate phase from the water phase. The ethyl acetate was evaporated to yield an organic product which was purified by chromatography (silica preparative plate eluted three times with 60% ethyl acetate-hexane) to yield 18 mg of an organic compound which was then dissolved in and crystallized from methanol to give 6 mg of substantially pure material having a melting point of 235-239°C. The organic compound was identified by NMR as 3β,16α-dihydroxy-Δ5-androstene-7,17-dione (6). The results of the NMR analysis are set forth in Table Eleven.

### Example IX (Steroid 7)

### Synthesis Δ5-Androstene-3β-propionoxy-16β-acetoxy-7,17-dione

*(Step 1)* Into a round bottom flask equipped with a magnetic stirrer was placed 1.52 mmoles of 3β-propionoxy-16α-hydroxy-Δ5-androstene-7,17-dione, 3.0 mmoles of triphenylphosphine, 6.0 mmoles of acetic acid in 11 ml of tetrahydrofuran. This first mixture was placed under N₂ and cooled to 0°C in an ice bath. To the first mixture was added 3.0 mmoles of diethyl azodicarboxylate dropwise to form a second mixture. The second mixture was warmed to 25°C and maintained at this temperature for 18 hr. It was then partitioned between ether-hexane and water. The ether-hexane layer was washed with water, water, saturated NaHCO₃ aqueous solution, saturated NaC1 aqueous solution, dried over Na₂SO₄ and evaporated to give an organic solid which was purified by chromatography on 130 gm silica eluted with ethyl acetate-hexane (20:80 to 50:50) with 25 ml fractions and crystallized from dichloromethane-hexane to yield 0.267 gm of an organic compound. The organic compound was identified by NMR as 3β-propionoxy-16β-acetoxy-Δ5-androstene-7,17-dione containing ~ 20% of the 16α epimer. The results of the NMR analysis are set forth in Table Twelve.

*(Step 2)* Conversion to Δ5-androstene-3β,16β-dihydroxy-7,17-dione can be done as described in Example VIII.

### Example X (Steroid 8)

### Synthesis Δ5-Androstene-3β,7α,17β-triol-16-one

*(Step 1)* Into a 100 ml round bottomed flask was placed 3 grams (7.7 mmoles) of 3β,16α-diacetoxy DHEA (prepared in accordance with the procedure set forth in Numazawa, M. and Osawa, Y. Steroids, vol. 32, p 519 (1978)) and 3.5 grams NaHCO₃ in 50 ml of hexane to form a first mixture. The first mixture was stirred and heated to reflux under a N₂ atmosphere. To the first mixture was added 1.6 grams dibromantin (1,3-dibromo-5,5-dimethylhydantoin) to form a second mixture.

(Step 1) The second mixture was stirred, refluxed for 30 minutes, and then cooled to room temperature. The refluxed second mixture was filtered to remove solids and washed with CH₂Cl₂. The resultant filtrate was concentrated to near dryness in vacuo using a water bath maintained below 35°C.

*(Step 3)* The dried filtrate was resolubilized in 21 ml of toluene in a one liter stoppered flask equipped with a magnetic stirrer and placed in an ice bath. Into the resolubilized filtrate was added 2.1 grams anhydrous LiBr in 80 ml ice-cold acetone to form a third mixture. The third mixture was shielded from light and stirred continuously for three hours at 0°C. The resulting mixture containing predominantly 7α-bromo was used immediately in step four.

*(Step 4)* Into a 500 ml flask equipped with a magnetic stirrer was placed 80 ml dichloromethane, 21 ml glacial acetic acid, and 6.7 grams of silver acetate to form a first suspension. The first suspension was stirred continuously for 20 minutes at room temperature. The stirred first suspension was added under constant agitation into the warmed third mixture to form a second suspension. The second suspension was constantly stirred for 30 minutes at room temperature after which the suspension was filtered through a sintered glass funnel to separate a solid residue. The filtrate was concentrated to yield an oily residue.

*(Step 5)* To the oily residue was added 300 ml H₂O and sufficient NaHCO₃ to achieve a fourth mixture with a neutral pH. The fourth mixture was extracted five times with 150 ml of ethyl acetate, the organic layers combined, washed with brine, dried over MgSO₄ and concentrated to dryness.

(Step 6) The crude organic phase was separated by chromatography (silica gel eluted with ethyl acetate : pet ether at 1:3, 1:2 and 1:1) to yield 700 mg (20%) of a first organic fraction and 1.5 grams (48%) of a second organic fraction. After crystallization from diethyl ether the first (mp 170-172 °C) and second (mp 155-158 °C) organic fractions were identified by NMR as Δ5-androstene-3β,7α,16α-triacetoxy-17-one and the corresponding Δ5-androstene-3β,16α-diacetoxy-7α-ol-17-one (83) respectively. The results of the NMR analysis of these two compounds are set forth in Tables Thirteen and Fourteen.

(Step 7) Into a flask equipped with a magnetic stirrer was added 400 mg (1 mmole) of the Δ5-Androstene-3β, 16α-diacetoxy-7α-hydroxy-17-one (83) and 342 mg of K₂CO₃ in 25 ml of methanol at room temperature to form a fifth mixture. The fifth mixture was stirred for two hours. The alkaline solution enolized the 17-keto to form the more stable 17-hydroxy-16-one compound. Δ5-Androstene-3β,7α,17-trihydroxy-16-one (8) was isolated from the fifth mixture by filtering to remove the insoluble salt, evaporating the methanol in vacuo, purifying the organic residue over silica gel and crystallizing the organic compound from methanol-ethyl ether solution. The crystallized organic fraction (180 mg, 56%; mp:>230°C) was identified by NMR as Δ5-androstene-3β,7α,17-trihydroxy-16-one (8). The results of the NMR analysis are set forth in Table Fifteen.

### Example XI (Steroid 9)

### Synthesis A5-Androstene-3β,17β-diol-7.16-dione

(Step 1) Into a round bottom flask equipped with a magnetic stirrer was added 1.05 grams (2.80 mmoles) Δ5-androstene-3β-propionoxy-16α-hydroxy-7,17-dione (63), 80 ml methanol and 40 ml of a 5% aqueous Na₂CO₃ solution while stirring rapidly to form a first mixture. The first mixture was stirred for 42 hours after which the methanol was evaporated and a combination of 100 ml of water and 2 ml of acetic acid added to form a second mixture. A solid material was filtered from the second mixture, resolubilized in methanol and then crystalized to yield 0.324 gm of an organic compound.

A small sample of the organic compound was again recrystalized from methanol to produce a purified sample having a melting point of 215-218°C. The first organic compound was identified by NMR as Δ5-androstene-3β,17β-diol-7,16-dione. The results of the NMR analysis are set forth in Table Sixteen.

**Table One**

| NMR Results | | |
|---|---|---|
| 3β-acetoxy-17-hydroxy-Δ5,Δ16-androstadiene-7-one-trimethylsilyl ether (52a) | | |
| Conditions | Peak | Significance |
| (δ) CDCl₃ 270 MHz | 0.21 | s, O-SiMe₃ |
| | 0.87 | s, 18-CH₃ |
| | 1.25 | s, 19-CH₃ |
| | 2.07 | s, CH₃COO |
| | 2.75 | ddd, J=14,6,3, Hz 15α-H |
| | 4.54 | dd, J=3,1.5 Hz 16-H |
| | 4.73 | tt, J=11,5 Hz 3α-H |
| | 5.76 | d, J=2 Hz 6-H |

**Table Two**

| NMR Results | | |
|---|---|---|
| 3β-(trimethylsilyl)acetoxy-17-hydroxy-Δ5,Δ16-androstadiene-7-one trimethylsilyl ether (52b) | | |
| Conditions | Peak | Significance |
| (δ) CDCl₃ 270 MHz | 0.13 | s, C-SiMe₃ |
| | 0.21 | s, O-SiMe₃ |
| | 0.87 | s, 18-CH₃ |
| | 1.25 | s, 19-CH₃ |
| | 1.90 | s, CH₂-Si |
| | 2.75 | ddd, J=14,6,3, Hz 15α-H |
| | 4.54 | dd, J=3,1.5 Hz 16-H |
| | 4.73 | tt, J=11,5 Hz 3α-H |
| | 5.76 | d, J=2 Hz 6-H |

**Table Three**

| NMR Results | | |
|---|---|---|
| 3β-acetoxy-16α-phenylseleno-Δ5-androstene-7,17-dione (53a) | | |
| Conditions | Peak | Significance |
| (δ) CDCl₃ 270 MHz | 0.92 | s, 18-CH3 |
| | 1.21 | s, 19-CH₃ |
| | 2.07 | s, CH₃COO |
| | 3.00 | ddd,J=14,5,0.5 Hz |
| | | 15α-H |
| | 4.10 | d, J=7.5 Hz J_{SeH}=5 Hz |
| | | 16β-H |
| | 4.73 | tt, J=11,5 Hz 3α-H |
| | 5.74 | d, J=1.5 Hz 6-H |
| | 7.31 | m, SePh |
| | 7.68 | m, SePh |

**Table Four**

| NMR Results | | |
|---|---|---|
| 3β-(trimethylsilyl)acetoxy-16α-phenylseleno-Δ5-androstene-7,17-dione (53b) | | |
| Conditions | Peak | Significance |
| (δ) CDCl₃ 200 MHz | 0.13 | s, C-SiMe₃ |
| | 0.91 | s, 18-CH₃ |
| | 1.21 | s, 19-CH₃ |
| | 1.91 | s, CH₂Si |
| | 3.00 | ddd,J=14 5.5,0.5 Hz 15α-H |
| | 4.10 | d, J=7.5 Hz J_{SeH}=5 Hz 16β-H |
| | 4.73 | tt, J=10.5 Hz 3α-H |
| | 5.74 | d, J=1.5 Hz 6-H |
| | 7.32 | m, SePh |
| | 7.68 | m, SePh |

**Table Five**

| NMR Results | | |
|---|---|---|
| 3β,16-diacetoxy-Δ5-androstene-16-phenylseleno-7,17-dione (55) | | |
| Conditions | Peak | Significance |
| (δ) CDCl₃ 200 MHz | 1.25 | s, 18-CH₃ |
| | 1.35 | s, 19-CH₃ |
| | 2.05 | s, 3-CH₃COO |
| | 2.09 | s, 16-CH₃COO |
| | 3.20 | dd,J=14.5,6 Hz 15α-H |
| | 4.73 | tt, J=11,5.5 Hz 3α-H |
| | 5.74 | d, J=1.5 Hz 6-H |
| | 7.35 | m, SePh |
| | 7.68 | m, SePh |

**Table Six**

| NMR Results | | |
|---|---|---|
| 3β,16-diacetoxy-Δ5,Δ15-androstadiene-7,17-dione (57) | | |
| Conditions | Peak | Significance |
| (δ) CDCl₃ 200 MHz | 1.24 | s, 18-CH₃ |
| | 1.30 | s, 19-CH₃ |
| | 2.07 | s, 3-CH₃COO |
| | 2.25 | s, 16-CH₃COO |
| | 4.75 | tt, J=11,5 Hz 3α-H |
| | 5.83 | d, J=1.5 Hz 6-H |
| | 7.82 | broad s, 15-H |

**Table Seven**

| NMR Results | | |
|---|---|---|
| 3β-acetoxy-A5-androstene-7,16,17-trione (5) | | |
| Conditions | Peak | Significance |
| (δ) CDCl₃ 270 MHz | 1.07 | s, 18-CH3 |
| | 1.30 | s, 19-CH₃ |
| | 2.05 | s, 3-CH₃COO |
| | 2.44 | dd,J=18.5,8 Hz 15β-H |
| | 2.61 | m, 4-H |
| | 3.43 | dd, J=18.5, 6.5 Hz 15a-H |
| | 4.75 | tt, J=11,5 Hz 3α-H |
| | 5.80 | d, J=1.5 Hz 6-H |

**Table Eight**

| NMR Results | | |
|---|---|---|
| 3β-propionoxy-A5-androstene-7,17-dione (61) | | |
| Conditions | Peak | Significance |
| (δ) CDCl₃ 270 MHz | 0.91 | s, 18-CH₃ |
| | 1.16 | t, J=7.5 Hz CH₃CH₂COO |
| | 1.25 | s, 19-CH₃ |
| | 2.15 | dt, J=19, 8.5 Hz 16α-H |
| | 2.33 | q, J=7.5 Hz CH₃CH₂COO |
| | 2.83 | dddd, J=15.5, 8.5, 4, 1 Hz 15α-H |
| | 4.75 | tt, J=11, 5 Hz 3α-H |
| | 5.77 | d, J=2 Hz 6-H |

**Table Nine**

| NMR Results | | |
|---|---|---|
| 3β-propionoxy-17-hydroxy-Δ5,Δ16-androstadiene-7-one trimethylsilyl ether (62) | | |
| Conditions | Peak | Significance |
| (δ) CDCl₃ 270 MHz | 0.18 | s, Si-Me₃ |
| | 0.85 | s, 18-CH3 |
| | 1.13 | t, J=7.5 Hz CH₃CH₂COO |
| | 1.21 | s, 19-CH₃ |
| | 2.14 | ddd, J=14.8, 10.7, 1.5 Hz 15β-H |
| | 2.31 | q, J=7.5 Hz CH₃CH₂COO |
| | 2.71 | ddd, J=14.8, 6.4, 2.1 Hz 15α-H |
| | 4.52 | dd, J=3.1, 1.5 Hz 16-H |
| | 4.72 | tdd, J=11.4, 5.3, 4.4 Hz |
| | 5.71 | 3α-H d, J=1.6 Hz 6-H |

**Table Ten**

| NMR Results | | |
|---|---|---|
| 3β-propionoxy-16α-hydroxy-Δ5 -androstene-7,17-dione (63) | | |
| Conditions | Peak | Significance |
| (δ) CDCl₃ 270 MHz | 1.00 | s, 18-CH₃ |
| | 1.15 | t, J=7.5 Hz CH₃CH₂COO |
| | 1.23 | s, 19-CH₃ |
| | 2.33 | q, J=7.5 Hz CH₃CH₂COO |
| | 2.75 | ddd, J=13.5, 6.5, 1.5 Hz 15α-H |
| | 4.42 | d, J=8 Hz 16β-H |
| | 4.76 | tt, J=10.5, 4.5 Hz |
| | 5.77 | 3α-H d, J=1.5 Hz 6-H |

**Table Eleven**

| NMR Results | | |
|---|---|---|
| 3β,16α-dihydroxy-Δ5-androstene-7,17-dione (6) | | |
| Conditions | Peak | Significance |
| (δ) CDCl₃ 270 MHz | 1.00 | s, 18-CH₃ |
| | 1.23 | s, 19-CH₃ |
| | 2.26 | ddd, J=14.5, 10.5, 8.6 Hz 15β-H |
| | 2.55 | ddd, J=14.1, 5.0, 1.3 Hz 4α-H |
| | 2.76 | ddd, J=14.6, 6.9 1.4 Hz 15α-H |
| | 3.69 | broad t, J=11 Hz 3α-H |
| | 4.41 | d, J=8.5 Hz 16β-H |
| | 5.75 | d, J=1.7 Hz 6-H |

**Table Twelve**

| NMR Results | | |
|---|---|---|
| 3β-propionoxy-16β-acetoxy-Δ5 -androstene-7,17-dione (7) | | |
| Conditions | Peak | Significance |
| (δ) CDCl₃ 270 MHz | 1.00 | s, 18-CH₃ |
| | 1.15 | t, J=7.5 Hz CH₃CH₂COO |
| | 1.25 | s, 19-CH₃ |
| | 2.12 | s, CH₃COO |
| | 2.33 | q, J=7.5 Hz CH₃CH₂COO |
| | 3.35 | ddd, J=11, 8.5 4 Hz 15α-H |
| | 4.74 | tt, J=11, 4 Hz 3α-H |
| | 5.77 | d, J=1.5 Hz 6-H |

**Table Thirteen**

| NMR Results | | |
|---|---|---|
| Δ5-Androstene-3β,16α-diacetoxy7α-hydroxy-17-one (83) | | |
| Conditions | Peak | Significance |
| (δ) CDCl₃ 300 MHz | 0.98 | s, CH₃ |
| | 1.02 | s, CH₃ |
| | 2.02 | s, 2xCO₂CH₃ |
| | 2.10 | s, CO₂CH₃ |
| | 4.70 | m, 1H, 3-H |
| | 5.02 | dd, 1H, J=2 Hz 7-H |
| | 5.40 | d, 1H, J=4 Hz 16-H |
| | 5.61 | d, 1H, J=2 Hz 6-H |

**Table Fourteen**

| NMR Results | | |
|---|---|---|
| Δ5-androstene-3β,16α-diacetoxy -7α-ol-17-one | | |
| Conditions | Peak | Significance |
| (δ) CDCl₃ 300 MHz | 0.97 | s, CH3 |
| | 1.02 | s, CH₃ |
| | 2.04 | s, CO₂CH₃ |
| | 2.15 | s, CO₂CH₃ |
| | 3.92 | m, 1H, 7-H |
| | 4.63 | m, 1H, 3-H |
| | 5.44 | d, 1-H, J=5 Hz 16-H |
| | 5.64 | d, 1-H, J=4 Hz 6-H |

**Table Fifteen**

| NMR Results | | |
|---|---|---|
| Δ5-androstene-3β,7α,17-trihydroxy-16-one (8) | | |
| Conditions | Peak | Significance |
| (δ) DMSO 300 MHz | 0.61 | s, CH₃ |
| | 0.92 | s, CH3 |
| | 3.32 | m, 3-H |
| | 3.60 | broad s 1H, 7-H |
| | 3.64 | s, 1H, 16-H |
| | 4.26 | d, 1H, J=2 Hz OH, D₂O exch. |
| | 4.68 | broad s, 1H OH, D₂O exch. |
| | 5.30 | broad s, 1H OH, D₂O exch. |
| | 5.42 | d, 1H, J=2 Hz 6-H |

**Table Sixteen**

| NMR Results | | |
|---|---|---|
| 3β,17β-dihydroxy-Δ5 -androstene-7,16-dione (9) | | |
| Conditions | Peak | Significance |
| (δ) CDCl₃ 270 MHz | 0.75 | s, 18-CH₃ |
| | 1.26 | s, 19-CH₃ |
| | 3.15 | ddd, J=19,7, 1.5 Hz 15α-H |
| | 3.72 | tt, J=10, 4.5 Hz 3α-H |
| | 3.78 | broad s 17α-H |
| | 5.77 | d, J=1.5 Hz 6-H |

## Claims

1. A steroid selected from the group consisting of
Δ5-Androstene-3β-acetoxy-7, 16, 17-trione
Δ5-Androstene-3β, 16α-dihydroxy-7, 17-dione
Δ5-Androstene-3β-propionoxy-16β -acetoxy-7, 17-dione
Δ5-Androstene-3β, 7α, 17β -triol-16-one
Δ5-Androstene-3β, 17β-diol-7, 16-dione
Δ5-Androstene-3β, 16α, 17β-triol, 7-one
and derivatives thereof wherein one or more of the hydroxyl or keto substituents is a group convertible thereto by hydrolysis, for use as weight control agent, especially for weight gain prevention, weight reduction and treatment of obesity.

2. A steroid according to claim 1 characterised in that it is intended for treating a human.

## Patentansprüche

1. Steroid, ausgewählt aus der Gruppe umfassend
Δ5-Androsten-3β-acetoxy-7, 16, 17-trion
Δ5-Androsten-3β, 16α-dihydroxy-7, 17-dion
Δ5-Androsten-3β-propionoxy-16β-acetoxy-7, 17-dion
Δ5-Androsten-3β, 7α, 17β-triol-16-on
Δ5-Androsten-3β, 17β-diol-7, 16-dion
Δ5-Androsten-3β, 16α, 17β-triol-7-on
und Derivate derselben, wobei ein oder mehrere der Hydroxyl- oder Keto-Substituenten eine Gruppe sind, die durch Hydrolyse in diese umwandelbar ist, zur Verwendung als Gewichtskontrollmittel, insbesondere zur Verhinderung der Gewichtszunahme, zur Gewichtsverminderung und zur Behandlung der Fettsucht.

2. Steroid nach Anspruch 1, dadurch gekennzeichnet, daß dieses zur Behandlung eines Menschen bestimmt ist.

## Revendications

1. Un stéroïde choisi dans la classe formée par
la Δ5-androstène-3β-acétoxy-7,16,17-trione
la Δ5-androstène-3β,16α-dihydroxy-7,17-dione
la Δ5-androstène-3β-propionoxy-16β-acétoxy-7,17-dione la Δ5-androstène-3β,7α,17β-triol-16-one
la Δ5-androstène-3β,17β-diol-7,16-dione
la Δ5-androstène-3β,16α,17β-triol-7-one
et leurs dérivés dans lesquels un ou plusieurs des substituants hydroxyle ou céto est un groupe convertible en celui-ci par hydrolyse, pour son utilisation comme agent de régulation pondérale, notamment pour la prévention du gain de poids, la réduction du poids et le traitement de l'obésité.

2. Un stéroïde selon la revendication 1, caractérisé en ce qu'il est destiné à traiter un être humain.
